# EUROPEAN PATENT SPECIFICATION

(11) **EP 2 254 491 B1**
(45) Date of publication and mention of the grant of the patent: **24.08.2011**
(21) Application number: 09713810.1
(22) Date of filing: 19.02.2009
(51) Int. Cl.: A61B 17/72

(54) **A DEVICE FOR REDUCING A BONE FRACTURE**
VORRICHTUNG ZUR REPOSITION EINER KNOCHENFRAKTUR
DISPOSITIF PERMETTANT DE RÉDUIRE UNE FRACTURE D'OS

(30) Priority: 27.02.2008 IT VI20080046
(43) Date of publication of application: 01.12.2010
(73) Proprietor: Delta System S.R.L. - Società Unipersonale, 36100 Vicenza (VI) (IT)
(72) Inventor: CHEMELLO, Antonio, I-36100 Vicenza (VI) (IT); CHEMELLO, Paolo, I-33100 Udine (UD) (IT)
(74) Representative: Ziliotto, Tiziano
(86) International application number: PCT/IB2009/000320
(87) International publication number: WO 2009/106951

(56) References cited:
- WO-A-97/37606
- WO-A-2004/026158
- WO-A-2004/049963
- WO-A-2005/094706

## Description

The invention relates to orthopaedic and traumatological surgery.

In particular, the invention concerns the surgical treatment of bone fractures. More in detail, the invention relates to a device for reducing a bone fracture, also called an intramedullary nail, with improved functional characteristics by comparison with the nails of known type, for use in reducing bone fractures, and long bone fractures in particular.

It is common knowledge that surgery is a widely-used method for treating bone fractures, especially those involving the long bones. Surgery has ample means of synthesis available for reducing such fractures, including the intramedullary nail, which is being used more and more.

This device or so-called nail, for reducing a bone fracture comprises an elongated tubular body that is inserted inside the suitably-milled medullary canal of the fractured bone.

According to a known embodiment, the intramedullary nail comprises a set of anchoring elements, also called pins, contained inside the tubular body. These pins are designed to emerge through suitable openings provided on the tubular body in order to become implanted in the cortical part of the fractured bone, enabling the distal fixing of the nail to the bone.

The emergence of the pins is actuated by the surgeon with the aid of suitable tools, which are used to take action on actuating means comprising mechanical members inserted in the body of the nail.

At the end of the period of convalescence, the surgeon takes action again, using the above-mentioned tools, to make the pins withdraw completely inside the tubular body, enabling the device to be removed from the bone.

A first drawback of this type of device lies in that the procedures involved in making the pins emerge and withdraw are not safe and reliable.

More in particular, a drawback of this type of nail lies in that the aforesaid pins become caught against the nail during the installation and extraction of the nail.

This interferes, moreover, with the proper emergence and optimal withdrawal of the pins from/in the tubular body.

Another disadvantage lies in that the surgeon may be unable to make all the pins withdraw and some of them impact against the edge of the above-mentioned opening or seize up.

Another drawback, that is related to those mentioned above, lies in that a considerable effort is required on the part of the surgeon to govern the emergence and withdrawal of the pins.

A further drawback lies in that said effort may lead to the rupture of some of the pins, with the consequence that some of the resulting fragments remain inside the bone, possibly giving rise to clinical complications.

Another drawback relating to those previously mentioned lies in that surgeons are unable to take action in full control and in safe conditions, and they cannot be assured the sensitivity they need in the procedure to accurately control the position reached by the pins inside the bone.

The object of the present invention is to overcome the above-mentioned drawbacks.

A particular object of the invention is to realise a device for reducing bone fractures with improved functional features by comparison with those of the nails of known type, for use in reducing fractures of long bones, that enables the above-mentioned drawbacks to be overcome.

Another object is to realise a device suitable for being fixed distally to the cortical part of the fractured bone in a rapid and reliable manner.

Another object is to realise a device in which the procedure for withdrawing the pins inside the tubular body is rapid and reliable.

Another object is to realise a device in which any seizure of the pins is avoided during the procedure for extending them from or withdrawing them into the tubular body.

Another object is to realise a device that is more straightforward to implant and remove than comparable nails of known type.

Another object is to realise a device that facilitates the surgeon's work during the procedures for the installation and removal of the device, and particularly that gives the surgeon greater control over its positioning and fixing to the bone than with comparable nails of known type.

Another object is to realise a device in which the pins complete the above-described steps smoothly, without encountering any obstacles or any other problem that might prevent or restrict said action.

Another object is to realise a device in which the trajectories covered by the pins in order to emerge from the nail and become anchored to the cortical part of the bone, and subsequently to withdraw therefrom, are controlled and improved by comparison with the devices of known type. More in particular, an object is to ensure that the trajectories covered by the pins during their emergence and withdrawal coincide substantially with one another.

A last but not least object is to realise a device that is easy to manufacture and assemble, economical, and user-friendly, in which the procedures involved in fixing it distally to the bone and later withdrawing the pins used to do so are particularly straightforward and require no particular effort on the part of the surgeon.

The above-mentioned objects are achieved by a device for reducing a bone fracture as described and characterised in the main claim.

Advantageous embodiments form the object of the dependent claims.

The proposed solution advantageously enables a device to be realised in which the system that achieves its distal fixing to the bone by means of the emergence of the pins and their subsequent withdrawal into their respective seats is more reliable and safe than in the devices of known type.

The proposed solution equally advantageously enables a device to be realised that assures the surgeon the ability to achieve the emergence and withdrawal of said pins easily and smoothly, in a calibrated manner and with a greater sensitivity. This advantageously enables any need to use x-rays to identify the position of the pins to be reduced to a minimum, thereby safeguarding the patient's health.

The proposed solution equally advantageously enables a device to be realised in which the emergence and withdrawal of the pins takes place slidingly and with considerably less effort on the part of the surgeon than with comparable nails of known type. This also enables any risk of the rupture of said pins and the dispersion of the resulting fragments inside the bone to be avoided.

The above-mentioned objects and advantages are better illustrated in the description of several preferred embodiments of the invention, given here as non-limiting examples, with reference to the attached drawings, wherein:
- figure 1 shows a partial longitudinal sectional view of an embodiment of a the device according to the invention in a first working position;
- figure 1a shows an exploded view of a longitudinal section of the device in figure 1;
- figure 2 shows a partial longitudinal sectional view of the device in figure 1 in another working position, combined with a view thereof from above;
- figure 3 shows a side view of an element of the nail in figure 1;
- figure 4 shows another side view of the element in figure 3;
- figure 5 shows a cross-sectional view of the element in figure 3;
- figure 6 shows a side view of a variant of the element in figure 3;
- figure 7 shows another side view of the element in figure 6;
- figure 8 shows a longitudinal sectional view of another element of the nail in figure 1;
- figure 9 shows a further longitudinal sectional view and a cross-sectional view of the element in figure 8;
- figure 10 shows a longitudinal sectional view of another element of the nail in figure 1;
- figure 11 shows a cross-sectional view along Z-Z of the element in figure 10;
- figure 12 shows a side view of another element of the nail in figure 1;
- figure 13 shows a view from above of the element in figure 12;
- figure 14 shows a side view of another embodiment of the device according to the invention, applied to a bone with a simple fracture;
- figure 15 shows a side view of the device in figure 14 applied to a bone with a multifragmentary fracture;
- figures 16 and 17 show two partial longitudinal sectional views of two further embodiments of a device according to the invention;
- figure 18 shows a longitudinal sectional view of another embodiment of the device according to the invention, applied to a bone with a peri-/sub-trochanteric fracture:
- figure 19 shows a cross-sectional view along C-C of the device in figure 18;
- figure 20 shows a longitudinal sectional view of another embodiment of the device according to the invention, applied to a fracture of the neck of femur;
- figure 21 shows a longitudinal sectional view of several parts of the device in figure 20;
- figure 22 shows an exploded longitudinal sectional view of the parts shown in figure 21;
- figure 23 shows a side view of the key used by the surgeon to take action on the device according to the invention;
- figure 24 shows a longitudinal sectional view of the device in figure 1, to which the key in figure 23 is coupled during an installation step;
- figures from 24a to 24d each show an enlarged view of a partial longitudinal sectional view of several parts of the device in figure 1 in the various positions that they can occupy.

First of all, it should be noted that corresponding elements of the various embodiments are identified by means of the same numerical references. The position indicators mentioned in the various embodiments can be logically transferred to the new position, in the event of any change in position.

While the following description and the related figures illustrate certain particular embodiments of the present invention, this is on the understanding that the present invention is not limited to said particular embodiments, but rather that the particular embodiments described herein merely clarify certain aspects of the present invention, the object and scope of which are defined in the claims.

The embodiments of the invention described below refer to a device for reducing bone fracture, and more in particular to an improved intramedullary nail suitable for use in reducing bone fractures, and especially those of the long bones.

In particular, said device is preferably for use in reducing fractures of bones such as the humerus, tibia, femur, or intratrochanteric or peri-/sub-tronchanteric fractures, and those affecting the neck of femur.

Clearly, the device can also be used to treat other types of fracture that necessitate the implantation of nails serving a similar functional purpose to that of the device according to the invention.

More precisely, the device according to the invention is designed to be implanted inside the suitably-milled medullary canal of a fractured bone.

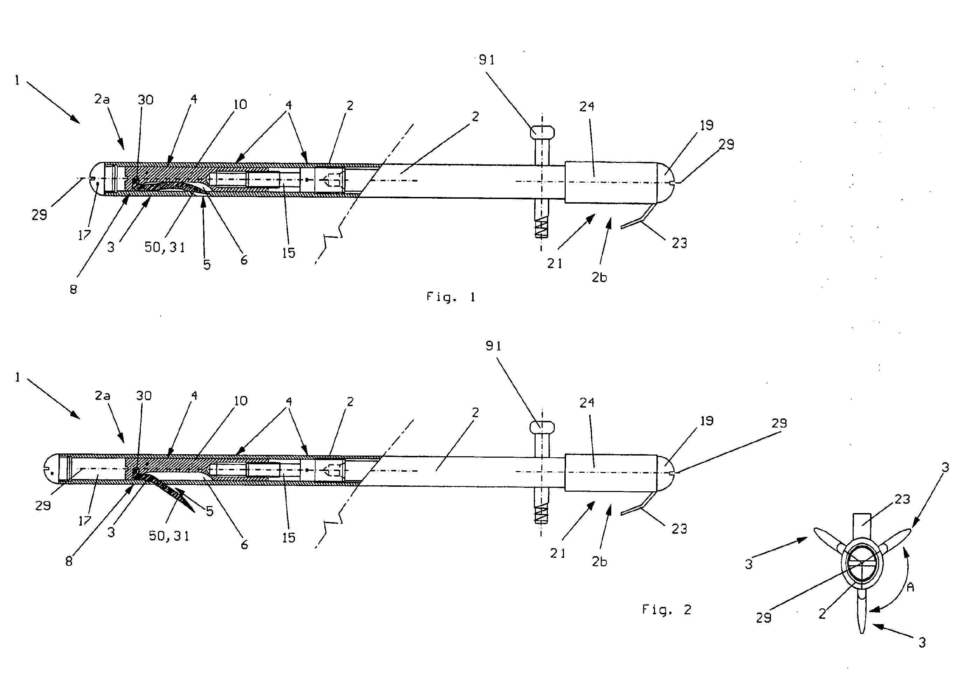

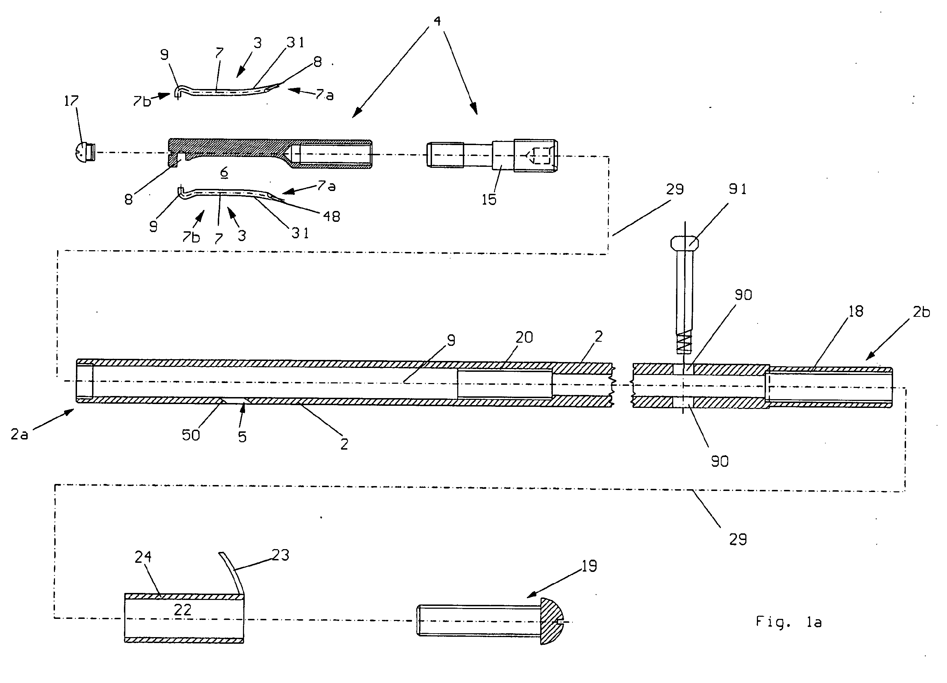

A preferred, non-limiting embodiment of the device for reducing a bone fracture according to the present invention is shown in figures 1, 1a and 2, where it has been globally indicated by the numeral 1.

It comprises a main body 2 and one or more anchoring elements 3, which are designed to be displaced using actuating means, indicated globally by the numeral 4, from a position at rest inside the main body 2, shown in particular in figure 1, to a working position, outside the main body 2, shown in figure 2.

In their position at rest, the anchoring elements 3 are contained in corresponding seats 6, visible in figures 1, 1a and 2, from where they emerge through openings 5 in the main body 2.

It is emphasised that, in the preferred but non-limiting embodiment of the invention illustrated herein, the anchoring elements 3 are employed in a number amounting to three and they are arranged radially, at an angle A corresponding to 120° from each other, around the longitudinal axis 9.

This is on the understanding, however, that the number of anchoring elements 3 may differ in other versions.

According to the invention, the device 1 also comprises elastic means that cooperate with the anchoring elements 3 to drive said anchoring elements 3 towards the outside of the main body 2 when the actuating means 4 are operated.

In the preferred, non-limiting embodiment illustrated herein, the elastic means consist of the anchoring elements 3 themselves, also called pins, that are made in this case of an elastic and/or shape-memory material.

More in particular, the anchoring elements 3, shown in figures from 3 to 5, comprise a shank 7 with a first end 7a and a second end 7b, and with means 9 for connecting them to the actuating means 4.

The shank 7 is shaped and has a length L and a height h.

The cross-section of the shank 7 is preferably circular, with a diameter D.

In the preferred, non-limiting embodiment illustrated herein, the shaped shank 7 is arched and substantially reproduces an arc of a circle. In other embodiments, the shank 7 substantially reproduces an arc of an ellipse or of a parabola.

The first end 7a terminates with a pointed terminal portion 48, or tip, suitable for being placed in contact with the inside wall of the bone and becoming implanted therein. The pointed terminal portion 48 is obtained by removing a part of the end 7a and particularly through the intersection of two spheres.

Clearly, the shape of the shank 7, and the radius of curvature R in particular, together with the features of the material of which the shank is made, determine the elastic characteristics of each element 3.

The above-mentioned elastic action is achieved when the elements 3, also called pins, are displaced from their position at rest into their working position.

Said action is such that each anchoring element 3 advantageously comes into contact with the bone as soon as possible.

Moreover, said elastic action means that each anchoring element 3 can acquire different conformations or shapes.

More in particular, each anchoring element 3 can acquire at least a first conformation or shape when it is in its resting position inside its seat 6, and at least a second conformation or shape.

Figure 1 shows a first conformation or shape that can be acquired by each element 3 when it occupies its a position at rest inside its seat 6.

It should be noted that each anchoring element 3 is forced into this first conformation, being compressed inside its corresponding seat 6. In fact, the edge 50 of the opening 5 interferes with the surface 31 of the element 3 that is consequently forced to become elastically deformed.

Each element 3 is arranged, moreover, so that its end 8 designed to engage in the bone O coincides with the corresponding opening 5 and does not extend from the main body 2 of the nail 1.

This advantageously enables a proper emergence of each anchoring element 3 to be guaranteed during the installation of the nail 1, preventing the element 3 from becoming caught in its corresponding seat 6, and thereby facilitating the implantation of each element 3 in the cortical tissue of the bone.

Figure 2 shows the second conformation or shape acquired by each element 3 when it is in its working position.

It should be noted, moreover, that in the particular embodiment shown herein, the same above-described elastic action is also exploited when the actuating means are used to bring the elements 3 from their working position back to their position at rest.

The connecting elements 9 consist of the second end 7b of each pin 3, which is suitably shaped to fit inside a corresponding cavity 8 visible in figures 1, 1a and 2, belonging to the aforesaid actuating means 4.

Said elements 9 enable each pin 3 to rotate around an axis 30 that is substantially orthogonal to the longitudinal axis 29 of the device 1, advantageously facilitating their radial emergence through the opening 5, as also described later on.

In the preferred but non-limiting embodiment of the invention discussed herein, the pins 3 have a global length L coming between 22 mm and 36 mm, and their end 7a has a radius of curvature R coming between 55 mm and 83 mm, and preferably amounting to 55.6 mm or 60 mm, or 66.5 mm, or 75 mm or 82.5 mm, while the diameter D of the shank 7 comes between 1.5 mm and 2 mm.

More in particular, in a preferred embodiment, the pins 3 have an overall length of 26.5 mm, their ends 7a have an angle of 25°, and the diameter of the main body 2 is 1.8 mm.

Clearly, the above-stated dimensions may need to differ in other embodiments of the present invention.

It should also be noted that the pins 3 are preferably made of a material that is compatible with the human body and that is elastically deformable.

More precisely, according to a preferred embodiment, the pins are obtained by a process of drawing and/or moulding of metal materials.

In particular, in the specific embodiment illustrated herein, the pins 3 are made using materials belonging to the family of stainless steels type AISI 316L. Clearly, however, other types of material, even non-metallic, that have similar features to those of the above-specified materials, e.g. ceramics or plastics, or sintered materials, can also be used to manufacture the pins 3.

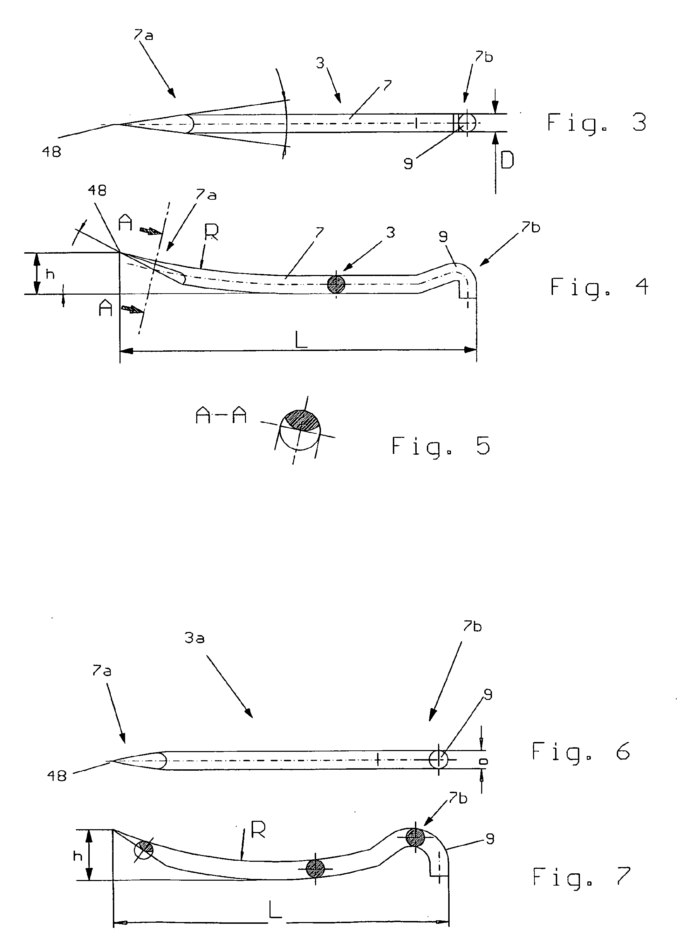

By way of example, figures 6 and 7 show a different embodiment of an anchoring element, globally indicated by the numeral 3a, with a different shape.

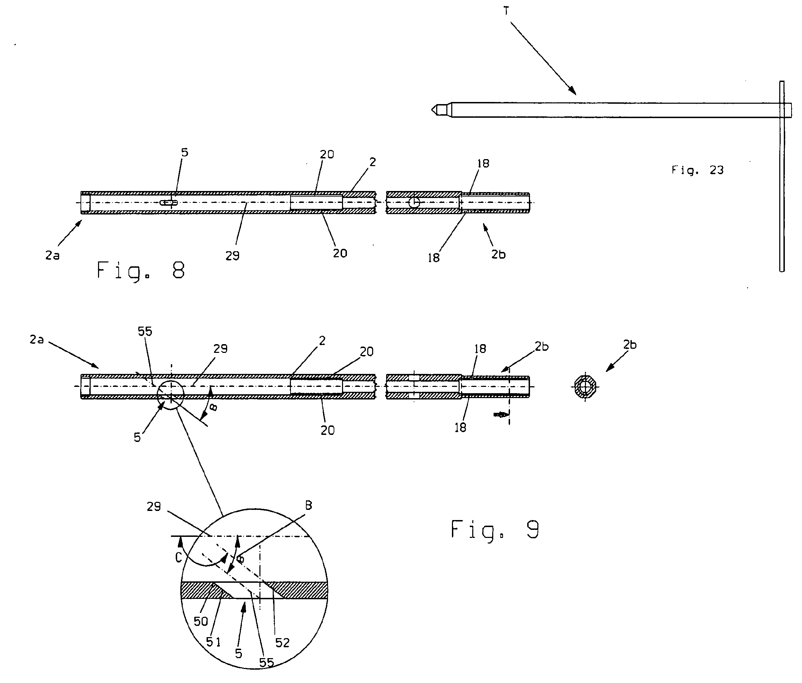

In the non-limiting embodiment illustrated herein, the main body 2 comprises a hollow body with a first and a second end, respectively indicated in figures 8 and 9 by the numerals 2a and 2b. More in particular, said hollow body comprises a tubular member with a preferably circular cross-section complete with the aforesaid through openings 5 around its lateral wall.

In the non-limiting embodiment illustrated herein, the main body also has a stretch at the upper end 2b that is substantially octagonal in shape externally, while the opposite, lower end 2a can be closed with a threaded plug 17, visible in figures 1, 1a and 2.

Said plug 17 is designed to serve as a limit stop and to stop any sliding of the actuating means 4 when they are operated.

In the preferred, non-limiting embodiment illustrated herein, there is a thread 18 on the inside of the tubular body 2, coinciding with the end 2b, on which a screw 19 engages for the purpose of juxtaposing the two stumps of fractured bone, as explained in more detail later on, and a thread 20 coinciding with the central portion of the tubular body 2 for coupling with a corresponding thread belonging, as we shall see better later on, to parts of the actuating means 4.

The nail 1 also comprises a cap 21, shown in figures 1, 1a and 2 with an axial hole 22 and a single or double arched appendage 23, suitable for resting against the outer wall of the bone.

The cap 21 is applied to the tubular body 2, engaging with a screw 19 in the thread 18 at the end 2b of the tubular body 2, through the axial hole 22 in said cap 21, enabling the proximal fixing of the nail 1 to the bone and the simultaneous compacting of the fractured stumps of bone.

The cap 21 also comprises a sleeve 24 with an octagonal cross-section and integral at its upper end with the appendage 23, designed to engage in the octagonally-shaped stretch of the end 2b.

This advantageously enables any accidental rotation of the fractured bone stumps to be prevented during the time it takes to reduce the fracture, since the above-described coupling between the cap 21 and the tubular body 2 prevents any mutual rotation between said two elements.

Moreover, said coupling advantageously enables a dynamic compacting of the fractured bone stumps, which are submitted to an axial load in that the cap 21 is capable of sliding on the end 2b of the tubular body 2 during the consolidation of the fractured bone.

It is worth noting that the body 2 advantageously had at least one through hole 90 lying crosswise and, more in particular, orthogonally to the longitudinal axis 29 of the body 2, that is visible, for instance, in figures 1, 1a and 2, each hole being designed to contain a corresponding anchoring screw 91, also called a cross screw, that can be used either in combination with the cap 21 or alone to achieve the proximal anchoring of the device 1.

In other words, according to a preferred embodiment, the cap can be substituted by the above-mentioned cross screw 91.

Said cross screw is particularly useful in the case of multi-fragmentary fractures. It should also be noted that said at least one through hole may have any shape of profile, and in particular it may be circular and/or elliptic, and/or polygonal, and/or mixtilinear, and may also be designed, as explained later on, to contain another device according to the intervention.

It is also worth noting that, in other embodiments of the present invention destined for use in reducing other types of fracture, the main body 2 may extend axially in a slightly arched conformation, or it may have a non-circular cross-section, such as a polygon, for instance, an ellipse or a mixtilinear cross-section. More in general, the shape of the body 2 substantially reproduces the shape in the longitudinal direction of the bone to which it is applied.

In the non-limiting embodiment illustrated herein, the through openings 5 are provided near the lower, or distal end 2a of the tubular body 2, to enable the distal attachment of the nail 1 to the inside wall of the bone, and they are preferably in the shape of a slot, as shown in figures 8 and 9.

It should be noted that, in the preferred embodiment illustrated herein, the walls of the opening 5 (through which the pins 3 move when they are actuated by the actuating means 4 in order to make them engage in the bone) preferably form an acute angle B with respect to the longitudinal axis 29 of the nail 1. More in particular, they tilt towards the upper, proximal end 2b and they form an angle preferably coming between 15° and 35° with the longitudinal axis 29 of the tubular body 2. Said type of pin 3 is also defined as *everted.*

More precisely, in the particular embodiment also illustrated in figure 9, the axis 55 of the openings 5 forms an angle B of 30° with the axis 9.

In another embodiment said angle B comes between 72° and 68°.

Other good result are reached with an angle B of 35°.

Other good result are reached with an angle B comes between 145° and 165°, and that preferably amounts to 150°.

Combined with the particular shape and size of the pins 3, and with the aforesaid elastic force, said solution also advantageously helps to facilitate the emergence of the pins from the corresponding openings 5 and the consequent anchoring of the pins 3 in the wall of the bone.

Said tilting of the wall 51 of the openings 5, visible in detail in the enlargement in figures 9, also advantageously enables the pins 3 to reach and penetrate the wall of the bone at a coupling angle designed to ensure a perfect, stable grip, with no movements or unwanted displacements.

This result is also assured by the particular conformation and curvature of the pins 3 and of their tips 8.

It should be noted that, in the preferred embodiment of the invention illustrated herein, the walls 52 of the openings 5 (through which the pins 3 move when they are actuated by the actuating means 4 to withdraw them from the bone), i.e. the walls closer to the proximal end, are also tilted with respect to the longitudinal axis 29 of the nail 1; in particular, they form an angle C with the longitudinal axis 29 of the tubular body 2 that comes between 145° and 165°, and that preferably amounts to 150°.

In another embodiment said walls 51, 52 form an angle with respect to the longitudinal axis 29 of said main body 2 that comes between 72° and 68°.

In another embodiment said walls 51, 52 form an angle with respect to the longitudinal axis 29 of said main body 2 that comes between 15° and 35°.

Other good result are reached with an angle of 35°.

This advantageously facilitates the withdrawal of the pins 3 inside their respective seats 6, when the surgeon removes the nail 1. Said solution also enables each pin 3 to slide substantially over the same trajectory both during their anchoring to the bone and during their withdrawal.

It should be noted that, in other embodiments of the present invention, the openings 5 may have different tilting angles providing they are suitable for achieving the previously-stated aims of the invention.

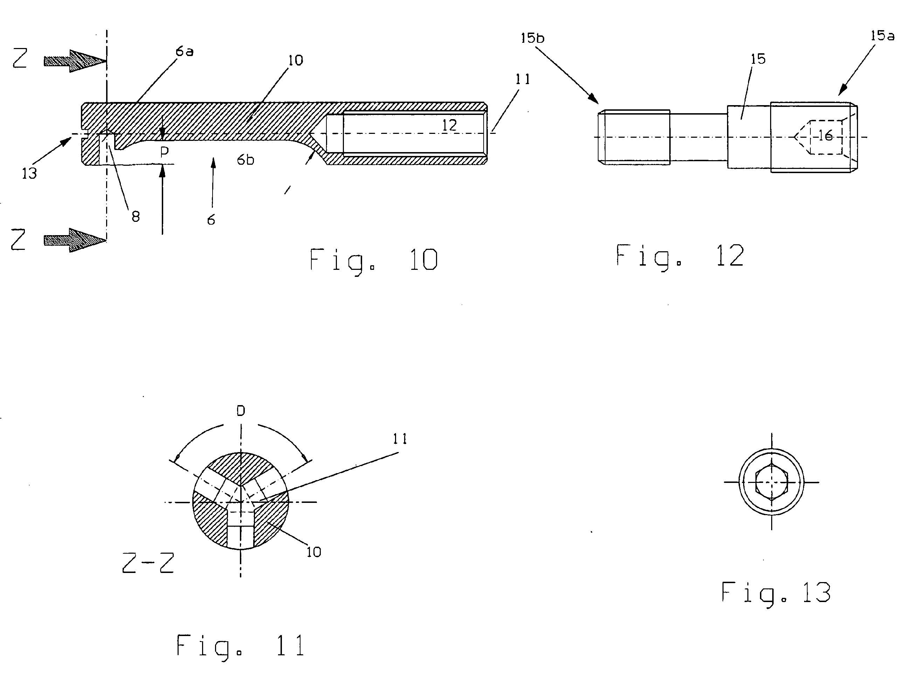

The actuating means 4, visible in figures 1, 1a and 2, are arranged inside the tubular body 2, and comprise a first element 10 co-operating with a second element 15 (also called a stud), shown respectively in figures 10, 11 and 12, 13. More in particular, in the preferred, non-limiting embodiment, the first element 10 is substantially cylindrical and designed to slide axially inside the main body 2.

It identifies a part of the seats 6 and is complete with a threaded axial hole 12 at one end, and a notch 13 at the opposite end, that is designed to contain the tip of a tool, which preferably consists of a screwdriver. During the assembly of the nail, the operator can take action, by suitably manoeuvring this screwdriver, to rotate and properly orient the element 10 so that the tips 8 of the pins 3 engage in the corresponding openings 5.

This advantageously enables the cylindrical element 10 to slide inside the tubular body 2 along the longitudinal axial 29, while preventing it from rotating around said axis.

The second element, or stud, 15 has a first end 15a and a second end 15b.

The first end 15a is complete with a first, preferably rightward-turning thread, designed to engage in the central threaded portion 20 of the tubular body 2.

The second end 15b is complete with a second thread turning in the opposite direction to the first and suitable for engaging in the threaded hole 12 in the cylindrical element 10.

In line with the end 15a, the stud 15 is also complete with an axial hole 16 that has a substantially hexagonal cross-section designed to contain the corresponding end of a tool preferably consisting of a long, slender hexagon-head wrench T, shown in figure 23, that is used by the surgeon - as explained in more detail later on - to take action on the actuating means 4.

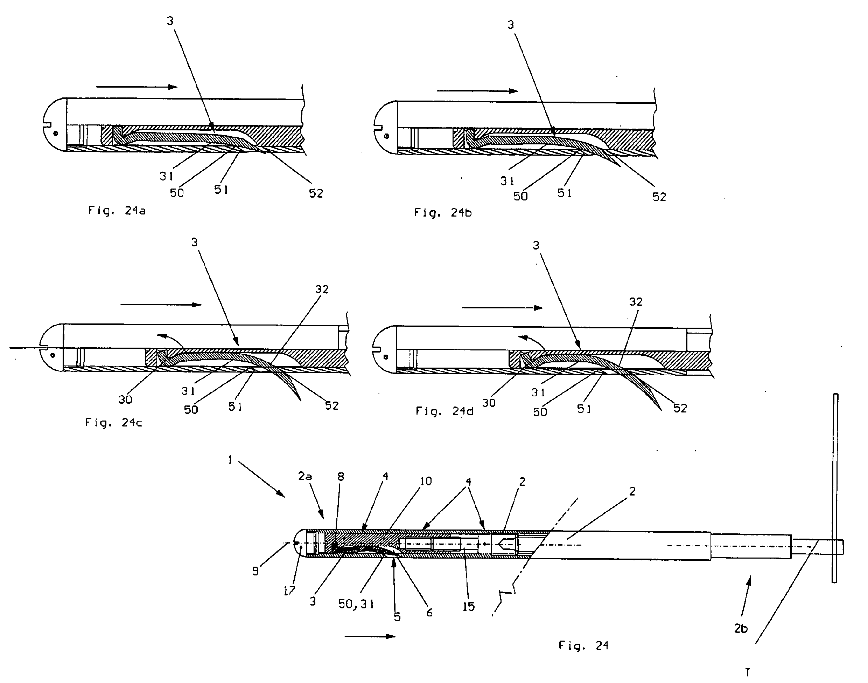

More in particular, by taking action on said wrench T as shown in figure 24, the surgeon is able to make the stud 15 rotate and thereby obtain a displacement of the cylindrical element 10 along the longitudinal axis 9, in one direction or the other, depending on the sense of rotation of the stud 15.

More precisely, by turning the stud 15 anticlockwise, the surgeon determines a displacement of the stud, and the consequent entrainment of the cylindrical element 10, towards the upper end 2b of the tubular body 2, thereby inducing the emergence of the pins 3.

Vice versa, by turning the stud 15 in a clockwise direction, the surgeon makes the pins 3 withdraw into their respective seats 6.

As concerns the seats 6, it should be noted that they are created longitudinally on the outer surface of the cylindrical element 10.

To be more precise, they comprise a first portion 6a designed to contain the end 7b of the corresponding pin 3, and a second portion 6b suitable for containing the remainder of the pin 3.

It should be noted, in particular, that the cavity 6b has a depth P, or height, that is less than the length, or height h, of the pin 3 that it contains, and this guarantees that the pin 3 is compressed when located inside the seat 6.

More in particular, said depth P preferably comes between 2.2 mm and 3 mm, and amounts to 2.2 mm in the particular embodiment illustrated herein.

It should be noted that said depth may have a different dimension in other embodiments of the invention, providing that it is suitable for achieving the previously-stated aims of the invention.

More in general, said depth depends not only on the height h of the pin that it must contain, but also on the elastic characteristics of said pin.

It should also be noted that, in the non-limiting embodiment of the invention illustrated herein, the number of seats 6 coincides with the number of pins 3, and more in particular it amounts to a number of three. The seats 6 are also arranged at equidistant angles around the longitudinal axis 11 of the cylindrical element 10, at an angle D of 120°, as shown in figure 8.

Clearly, in other embodiments of the present invention, there may also be a different number of seats 6, providing it coincides with the number of the pins 3, and of the openings 5, and these may be suitably arranged, providing they can achieve the previously-stated aims of the present invention.

Another variant of the invention differs from the previously-described embodiment in that each anchoring element 3 is moved into its second conformation by the action of members that take effect by means of direct contact with said element. More in particular, in a first solution, said members comprise a sloping plane against which each element comes into contact when the surgeon uses the actuating means 4.

Another variant of the invention differs from the previously-described embodiment in that the elastic means preferably comprise one or more springs that have one end in contact with a wall of the seat 6 and the other abutting against the pin 3.

Another variant of the invention differs from the previously-described embodiment in that the proximal fixing of the nail 1 is achieved by means of the cross screw 91, which engages in the crosswise hole 90 in the device 1.

Said embodiment is particularly suitable for use in the case of multifragmentary fractures, to prevent the displacement of the device due to muscle contraction and the consequent disruption of the fracture.

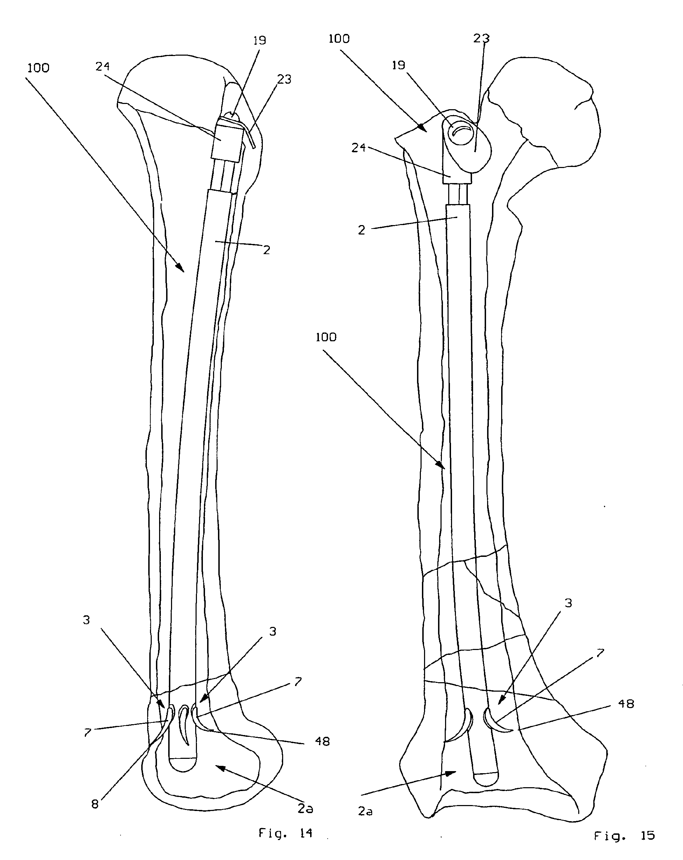

Another embodiment of the present invention, identified globally by the numeral 100 in figures 14 and 15, differs from the previous embodiments in that the pins are arranged so as to present their concavities facing in the opposite direction to the previously-described solutions, i.e. with their concavities not facing the axis of the nail 1 (also called *inverted* pins).

In this case, the walls of the openings 5 slope in the opposite direction to those of the previous cases, i.e. they slope towards the so-called distal end 2a. Said walls are designed to contain the tip 8 of the corresponding shank 7 of the pin that, in this case, faces towards the lower end 2a of the main body 2.

It should be noted that this type of pin is particularly suitable for treating simple or multifragmentary fractures occurring in the vicinity of the distal portion of the bone O.

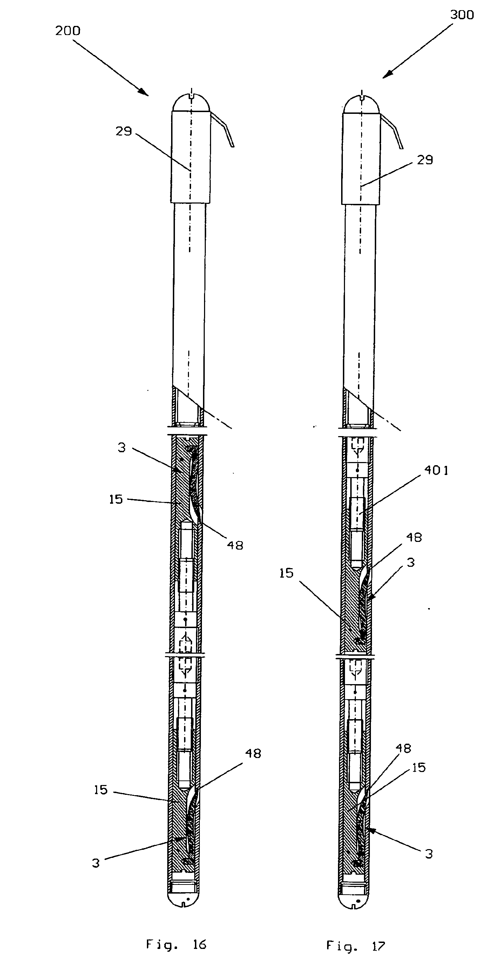

Another embodiment of the present invention, identified globally by the numeral 200 in figure 16, is distinguishable from the previously described embodiments in that it comprises two opposite sets of anchoring devices 3, with a space between them, along the longitudinal axis 29, with their respective tips 8 facing one another and oriented in a convergent direction with respect to the axis 29.

The anchoring elements 3 can each be actuated by a corresponding stud element 15. According to a preferred embodiment, the two elements 15 are also mechanically connected to one another to enable the surgeon to actuate the anchoring elements 3 simultaneously.

In this embodiment, the nail is said to be "double-acting" and it is particularly suitable for the treatment of multifragmentary fractures.

Another embodiment of the invention, identified globally by the numeral 300 in figure 17, differs again from the previously-described embodiments in that the anchoring elements 3 are arranged with their respective tips 8 not facing one another and oriented in a divergent direction with respect to the axis 29.

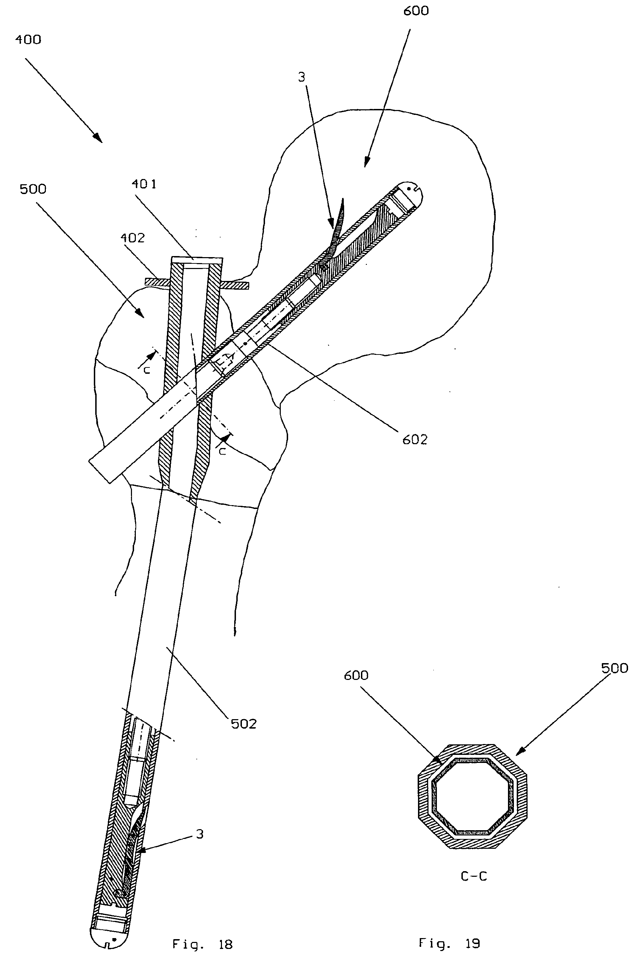

Another embodiment of the present invention, identified globally by the numeral 400 in figure 18, differs again from the previously-described embodiments in that it comprises two devices of the above-described type that cooperate with one another, indicated respectively by the numerals 500 and 600.

Said device 400 is particularly suitable for use in the treatment of the so-called peri-/sub-trochanteric fractures.

More in particular, this embodiment differs from those previously described in that at least a portion of the external profile of the device 600, shown in particular in figure 19, is polygonal or, more in general, mixtilinear. To be more precise, said portion coincides with the proximal end of the device 600 and advantageously enables the coupling of two devices, 500 and 600, preventing their mutual rotation in relation to their corresponding longitudinal axes.

It should be noted, moreover, that this last solution involves the use of a device 500 with a tubular body 502 that extends axially in a slightly curved shape designed to reflect the shape of the bone, and that it involves the insertion of a further device 600 designed to adhere, by means of the pins 3, to a part of the bone substantially at a far from negligible distance from the fracture.

It should also be noted that, in this case, the fracture is reduced by using two devices, one with everted pins 3 and the other with inverted pins 3.

It should further be noted that, in the above-mentioned solution, the device 402 prevents any rotation of the proximal part of the fracture, while any rotation between the two devices 500 and 600 is prevented by the presence of an octagonally-shaped connection, shown in figure 19.

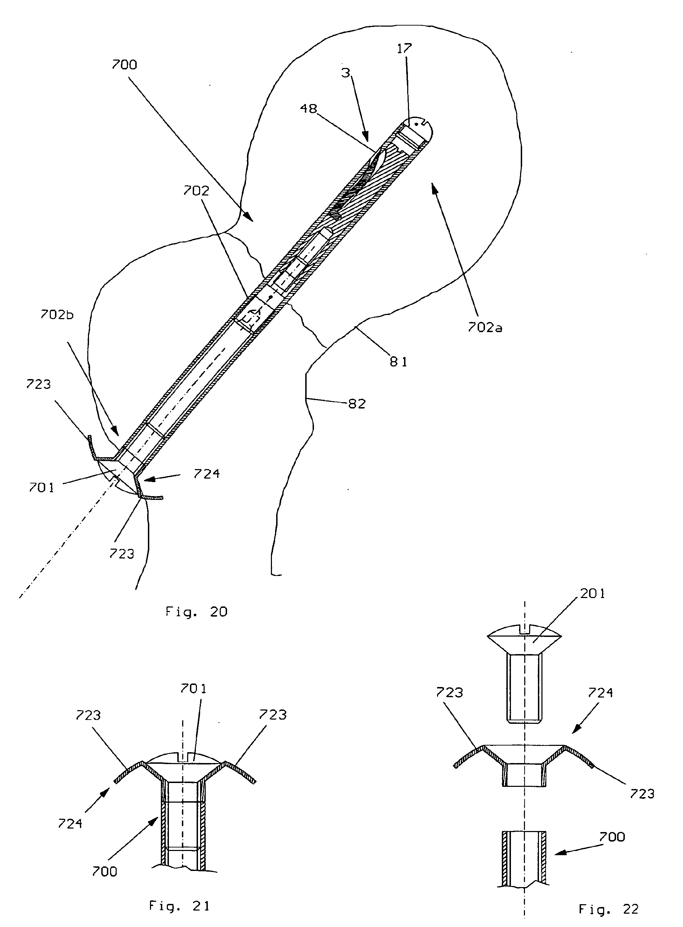

In another embodiment of the invention, shown in figure 20, a nail 700 is inserted in a fractured neck of femur.

It should be noted that the anchoring elements 3 of the device 700 are positioned in line with the part of the femur 81 coinciding with its maximum diameter. Said anchoring elements 3 have their tips 8 arranged towards the distal end 702a of the tubular body of the device 700.

The end 702b of the tubular body 702 is fixed to the stump 82 of the fractured bone by means of a screw 701, also shown in figures 21 and 22, which is first inserted through 30 a special sleeve 724 in the shape of an expansion washer with two symmetrical lips 723, then screwed onto the end 702b of the tubular body 702 of the nail 700.

Below is a description of how the nail of the invention functions, given with reference to the preferred embodiment illustrated in figure 1, there being no substantial differences with respect to the other embodiments described.

After making a hole in the patient's bone that needs to be reduced, the surgeon inserts the nail 1, configured as shown in figure 24, inside the medullary canal.

More in particular, the nail is configured with all the elements 3 contained and compressed in their respective seats 6, with their tips engaging in their respective openings 5, but not extend from the body 2, as shown in the enlargement in figure 24a.

The surgeon then takes action with the wrench to rotate the stud 15.

This makes the elements 3 move axially so that the point of contact between the surface 31 of each element 3 and the edge 50 of the opening 5 is displaced, thereby allowing each element 3 to return elastically to its natural shape.

In fact, the tip 8 of each pin 3 will naturally tend to move and emerge from the body 2, moving towards the wall of the bone, as shown in particular in figure 24b.

This solution advantageously prevents the anchoring elements 3, and their tips in particular, from coming into contact with the edge 52 of the opening 5 during the emergence of the pins 3 through their respective openings. In fact, the aforesaid elastic force drives the end of the pin to engage with the bone, facilitating said action.

By continuing to take action on the wrench T, the surgeon ensures that the pins 3 extend further outwards, moving them along the longitudinal axis 29 of the device 1 until a rotation of each pin 3 is obtained with respect to the axis 30 lying crosswise to the longitudinal axis 29 of the device 1.

Moreover, the surface 32 of each pin 3 comes into contact with the other wall of the opening 5, identified by the numeral 52. This advantageously guides the pin 3 along its trajectory to become implanted in the cortical layer of the bone, exploiting the elastic features of the pin.

This advantageously facilitates the emergence of the pins 3.

It also enables the angle of incidence between each pin and the cortical bone to be controlled, thereby improving the efficacy of the connection.

Finally, after anchoring the pins 3, the surgeon removes the wrench T and installs the cap 21 or cross screw 91, fixing it to the bone.

To remove the device, the surgeon completes the above-described procedure in reverse order. In this case, the elastic action facilitates the withdrawal of each pin 3.

In other words, each anchoring element 3 is designed to move elastically from a first position at rest into a second working position, also facilitated in doing so by its rotation around the crosswise axis 30.

The natural tendency of each anchoring element to return elastically to its original shape facilitates both the emergence from and its withdrawal inside the device 1.

In fact, as soon as its tip reaches the opening 5, the pin tends naturally to regain its original shape, so the tip 8 extends from the main body 2 and, in particular, it goes beyond the thickness of the edge of the main body surrounding the opening 5. This ensures that the anchoring element 3 emerges from its seat 6 without its tip 8 being able to reach the opposite edge 52 of the opening, and consequently risk interfering with the emergence of the pin 3.

The elements 3 thus emerge smoothly and slidingly, in a radial direction, from the tubular body 2 through their corresponding openings 5 to become implanted in the inside wall P of the bone O, without catching and/or seizing, and/or encountering any obstacles, and/or anything else that might prevent or restrict this action.

This also advantageously enables the pins 3 to slide and slip effectively through the openings 5 to withdraw inside their seats 6 when the surgeon takes action on the actuating means 4 during the removal of the nail 1.

It should be noted, moreover, that the distal fixing of the device achieved by means of the pins that become anchored to the fractured bone stump makes it unnecessary to take any x-rays to identify the hole for containing the fixing cross screw, as in the case of the nails of conventional type.

Although the invention has been described with reference to the attached drawings, it may undergo modifications during the implementation stage, all forming part of the same inventive concept expressed by the following claims and consequently equally protected by the present patent.

It is also emphasised that, where the characteristics mentioned in the following claims are followed by reference signs, these are given merely to facilitate the reader and shall not be intended as limiting the interpretation of said claims in any way.

It is also emphasised that all the details of the invention may be replaced by other technically equivalent elements, and that the materials used may be any, according to need, providing they are compatible with the contingent usage, and the same applies to the dimensions of the various elements.

## Claims

1. A device for reducing a bone fracture (1, 100, 200, 300, 400, 500, 600, 700) comprising a main body (2) and one or more anchoring elements (3) designed to be displaced by actuating means (4) from a position at rest inside respective seats (6) of said main body (2) to a working position outside said main body (2), wherein said one or more anchoring elements (3) are pushed outside said respective seats (6) of said main body (2) through a corresponding opening (5) when they are activated by said actuating means (4); **characterized in that** said anchoring elements (3) are made of an elastic material, and **in that** said seats (6) comprise a portion (6b) suited to contain a part of a corresponding anchoring elements (3), said portion (6b) having a depth (P), or height, that is less than the length, or height (h), of said corresponding anchoring elements (3) so that in said position at rest, each of said one or more anchoring elements (3) is compressed inside its corresponding seat (6) so as to acquire a first shape; and **in that** said anchoring elements (3) acquire a second shape differing from said first shape when they are moved to said working position outside said body (2).

2. A device according to claim 1), **characterized in that**, in said position at rest, each of said one or more anchoring elements (3) is arranged so that its end designed to become implanted in the bone (O) enters said opening (5) but does not extend from said main body (2).

3. A device (200, 300) according to one of claims 1) and 2), **characterized in that**, during the emergence/withdrawal of said one or more anchoring elements (3) from/in said seat (6), a surface (31, 32) of said anchoring element (3) comes into contact with a wall (50, 51, 52) of said opening (5) to guide each of said one or more anchoring elements (3).

4. A device (200, 300) according to any of the previous claims, **characterized in that**, during the emergence of said one or more anchoring elements (3), they each rotate around an axis (30) lying crosswise to the longitudinal axis (29) of said device (1) to facilitate their emergence through said at least one opening (5).

5. A device according to any of the previous claims, **characterized in that** said anchoring elements (3) comprise a shank (7) with a first end (7a) with a pointed terminal portion (48) suitable for becoming implanted in the bone, and a second end (7b) complete with means (9) for connecting said actuating means (4), said shank being shaped and having a length (L) and a height (h).

6. A device according to claim 5), **characterized in that** said shaped shank (7) is arched and substantially reproduces an arc of a circle, or an arc of an ellipse, or an arc of a parabola.

7. A device according to claims 5) or 6), **characterized in that** said means of connection (9) consist of one end (7b) of each anchoring element (3), said end being suitably shaped so as to fit inside a corresponding cavity (8) in said actuating means (4).

8. A device according to any of the previous claims, **characterized in that** said actuating means (4) comprise a first element (10) designed to slide axially inside said main body (2) mechanically connected to a second element (15) with a first thread suitable for coupling with a corresponding thread provided in said main body (2), said second element (15) being designed to be rotated by means of a tool (T).

9. A device according to claim 8), **characterized in that** at least a part of said seats (6) are created longitudinally on the outer surface of said first element (10) and comprise a first portion (6a) suitable for housing one end (7b) of said at least one anchoring element (3).

10. A device according to any of the previous claims, **characterized in that** said openings (5) are slots surrounded by walls (51, 52).

11. A device according to claim 10), **characterized in that** said walls (51, 52) form an angle with respect to the longitudinal axis (29) of said main body (2) that comes between 72° and 68°.

12. A device according to claim 10), **characterized in that** said walls (51, 52) form an angle with respect to the longitudinal axis (29) of said main body (2) amounts to 35°.

13. A device according to claim 10), **characterized in that** said walls (51, 52) form an angle with respect to the longitudinal axis (29) of said main body (2) that comes between 145° and 165°, and preferably amounts to 150°.

14. A device according to any of the previous claims, **characterized in that** the axis (55) of said openings (5) preferably forms an acute angle (B) with respect to the longitudinal axis (29) of said main body (2).

15. A device according to claim 14), **characterized in that** said angle (B) comes between 72° and 68°.

16. A device according to claim 14), **characterized in that** said angle (B) amounts to 35°.

17. A device according to claim 14), **characterized in that** said angle (B) comes between 15° and 35° and preferably amounts to 30°.

18. A device according to any of the previous claims, **characterized in that** said one or more anchoring elements (3) are arranged radially at an angle (A) amounting to approximately 120° from one another with respect to the longitudinal axis (9) of said main body (2).

19. A device according to any of the previous claims, **characterized in that** said main body (2) comprises a hollow body with said openings (5) and with a first and a second end (2a, 2b).

20. A device according to any of the previous claims, **characterized in that** said main body (2) has at least one stretch with a substantially polygonal external shape.

21. A device according to any of the previous claims, **characterized in that** it also comprises a cap (21) suitable for being placed on the external wall of the bone and anchorable to said bone.

22. A device according to any of the previous claims, **characterized in that** it also comprises a crosswise through hole (90) suitable for containing a screw (91) for anchoring to said bone and/or another device (1).

23. A device according to claim 22), **characterized in that** said crosswise through hole (90) has a circular and/or elliptic, and/or polygonal, and/or mixtilinear cross-section.

## Patentansprüche

1. Eine Vorrichtung zur Reduzierung eines Knochenbruchs (1, 100, 200, 300, 400, 500, 600, 700), einen Hauptkörper (2) umfassend sowie ein oder mehrere Verankerungselemente (3), die darauf ausgerichtet sind, durch Betätigungsmittel (4) aus einer Ruheposition innerhalb der jeweiligen Sitze (6) im Hauptkörper (2) in eine Arbeitsposition außerhalb des besagten Hauptkörpers (2) bewegt zu werden, wobei das besagte eine oder die mehreren Verankerungselemente (3) durch eine entsprechende Öffnung (5) aus den besagten, jeweiligen Sitzen 6) des Hauptkörpers (2) geschoben werden, wenn sie durch die besagten Betätigungsmittel (4) betätigt werden, **dadurch gekennzeichnet, dass** die besagten Verankerungselemente (3) aus einem elastischen Material bestehen, sowie **dadurch**, dass die besagten Sitze (6) einen Abschnitt (6b) umfassen, der geeignet ist, einen Teil eines entsprechenden Verankerungselements (3) zu enthalten, wobei der besagte Abschnitt (6b) eine Tiefe bzw. Höhe (P) aufweist, die geringer ist als die Länge bzw. Höhe (h) der besagten Verankerungselemente (3), so dass in der besagten Ruheposition das besagte eine bzw. jedes der mehreren Verankerungselemente (3) in seinen entsprechenden Sitz (6) gedrückt wird/werden, um eine erste Form anzunehmen, und des Weiteren **dadurch**, dass die besagten Verankerungselemente (3) eine zweite, von der ersten abweichende Form annehmen, wenn sie in die Arbeitsposition außerhalb des besagten Körpers (2) bewegt werden.

2. Eine Vorrichtung gemäß Patentanspruch 1), **dadurch gekennzeichnet, dass** in der besagten Ruheposition das besagte eine bzw. jedes der mehreren Verankerungselemente (3) so angeordnet ist/sind, dass sein Ende, das dazu bestimmt ist, in den Knochen (O) implantiert zu werden, in die Öffnung (5) einfährt, aber nicht aus dem besagten Hauptkörper (2) herausragt.

3. Eine Vorrichtung (200, 300) gemäß Patentanspruch 1) oder 2), **dadurch gekennzeichnet, dass** während des Herausfahrens/Zurückziehens des einen oder der mehreren Verankerungselemente (3) aus dem/in den besagten Sitz (6) eine Fläche (31, 32) des besagten Verankerungselements (3) mit einer Wand (50, 51, 52) der besagten Öffnung (5) in Kontakt kommt, um das eine bzw. jedes der mehreren Verankerungselemente (3) zu führen.

4. Eine Vorrichtung (200, 300) gemäß eines jeden der vorstehenden Patentansprüche, **dadurch gekennzeichnet, dass** während des Herausfahrens des einen bzw. der mehreren Verankerungselemente (3) sich jedes derselben um eine Achse (30) dreht, die quer zur Längsachse (29) der besagten Vorrichtung (1) steht, um ihr Herausfahren durch die besagte wenigstens eine Öffnung (5) zu erleichtern.

5. Eine Vorrichtung gemäß eines jeden der vorstehenden Patentansprüche, **dadurch gekennzeichnet, dass** die besagten Verankerungselemente (3) einen Schaft (7) mit einem ersten Ende (7a) mit einem spitzen Endabschnitt (48) umfassen, der geeignet ist, in den Knochen implantiert zu werden, sowie ein zweites Ende (7b) nebst Mitteln (9) zur Verbindung der besagten Betätigungsmittel (4), wobei der besagte Schaft geformt ist und eine Länge (L) sowie eine Höhe (h) aufweist.

6. Eine Vorrichtung gemäß Patentanspruch 5), **dadurch gekennzeichnet, dass** der besagte, geformte Schaft (7) gebogen ist und im Wesentlichen einen Kreisbogen oder einen Ellipsenbogen oder einen Parabelbogen darstellt.

7. Eine Vorrichtung gemäß Patentanspruch 5) oder 6), **dadurch gekennzeichnet, dass** die besagten Verbindungsmittel (9) aus einem Ende (7b) jedes Verankerungselements (3) bestehen, wobei dieses Ende angemessen geformt ist, um in einen entsprechenden Hohlraum (8) in den besagten Betätigungsmitteln (4) zu passen.

8. Eine Vorrichtung gemäß eines jeden der vorstehenden Patentansprüche, **dadurch gekennzeichnet, dass** die besagten Betätigungsmittel (4) ein erstes Element (10) umfassen, das so ausgelegt ist, axial in den besagten Hauptkörper (2) zu gleiten, der mechanisch mit einem zweiten Element (15) verbunden ist, welches ein erstes Gewinde zur Zusammenpassung mit einer entsprechenden, zweiten Gewinde in dem besagten Hauptkörper (2) aufweist, wobei das besagte zweite Element (15) darauf ausgelegt ist, durch ein Werkzeug (T) gedreht zu werden.

9. Eine Vorrichtung gemäß Patentanspruch 8), **dadurch gekennzeichnet, dass** wenigstens ein Teil der besagten Sitze (6) in Längsrichtung an der Außenfläche des besagten ersten Elements (10) geschaffen sind und einen ersten Abschnitt (6a) umfassen, der geeignet ist, ein Ende (7b) des besagten, wenigstens einen Verankerungselements (3) aufzunehmen.

10. Eine Vorrichtung gemäß eines jeden der vorstehenden Patentansprüche, **dadurch gekennzeichnet, dass** die besagten Öffnungen (5) Langlöcher sind, die von Wänden (51, 52) umgeben sind.

11. Eine Vorrichtung gemäß Patentanspruch 10), **dadurch gekennzeichnet, dass** die besagten Wände (51, 52) bezüglich der Längsachse (29) des besagten Hauptkörpers (2) einen Winkel zwischen 72° und 68° bilden.

12. Eine Vorrichtung gemäß Patentanspruch 10), **dadurch gekennzeichnet, dass** die besagten Wände (51, 52) bezüglich der Längsachse (29) des besagten Hauptkörpers (2) einen Winkel von 35°bilden.

13. Eine Vorrichtung gemäß Patentanspruch 10), **dadurch gekennzeichnet, dass** die besagten Wände (51, 52) bezüglich der Längsachse (29) des besagten Hauptkörpers (2) einen Winkel zwischen 145° und 165° bilden, und zwar vorzugsweise von 150°.

14. Eine Vorrichtung gemäß eines jeden der vorstehenden Patentansprüche, **dadurch gekennzeichnet, dass** die Achse (55) der besagten Öffnungen (5) vorzugsweise einen spitzen Winkel (B) bezüglich der Längsachse (29) des besagten Hauptkörpers (2) bildet.

15. Eine Vorrichtung gemäß Patentanspruch 14), **dadurch gekennzeichnet, dass** der besagte Winkel (B) zwischen 72° und 68° liegt.

16. Eine Vorrichtung gemäß Patentanspruch 14), **dadurch gekennzeichnet, dass** der besagte Winkel (B) 35° beträgt.

17. Eine Vorrichtung gemäß Patentanspruch 14), **dadurch gekennzeichnet, dass** der besagte Winkel (B) zwischen 15° und 35° liegt und vorzugsweise 30° beträgt.

18. Eine Vorrichtung gemäß eines jeden der vorstehenden Patentansprüche, **dadurch gekennzeichnet, dass** eines oder mehrere der besagten Verankerungselemente (3) in einem Winkel (A) von circa 120° zueinander radial bezüglich der Längsachse (29) des besagten Hauptkörpers (2) angeordnet sind.

19. Eine Vorrichtung gemäß eines jeden der vorstehenden Patentansprüche, **dadurch gekennzeichnet, dass** der besagte Hauptkörper (2) einen Hohlkörper mit den besagten Öffnungen (5) sowie mit einem ersten und einem zweiten Ende (2a, 2b) umfasst.

20. Eine Vorrichtung gemäß eines jeden der vorstehenden Patentansprüche, **dadurch gekennzeichnet, dass** der besagte Hauptkörper (2) wenigstens einen Abschnitt mit einer im Wesentlichen mehreckigen äußeren Form aufweist.

21. Eine Vorrichtung gemäß eines jeden der vorstehenden Patentansprüche, **dadurch gekennzeichnet, dass** sie außerdem eine Kappe (21) umfasst, die geeignet ist, an der Außenwand des Knochens positioniert und an dem besagten Knochen verankert zu werden.

22. Eine Vorrichtung gemäß eines jeden der vorstehenden Patentansprüche, **dadurch gekennzeichnet, dass** sie außerdem eine quer verlaufende Durchgangsöffnung (90) umfasst, die geeignet ist, eine Schraube (91) zur Verankerung an dem besagten Knochen und/oder an einer anderen Vorrichtung (1) aufzunehmen.

23. Eine Vorrichtung gemäß Patentanspruch 22), **dadurch gekennzeichnet, dass** die besagte, quer verlaufende Durchgangsöffnung (90) einen kreisförmigen und/oder elliptischen und/oder mehreckigen und/oder gemischtlinigen Querschnitt aufweist.

## Revendications

1. Dispositif pour la réduction d'une fracture osseuse (1, 100, 200, 300, 400, 500, 600, 700) comprenant un corps principal (2) et un ou plusieurs éléments d'ancrage (3) conçus pour être déplacés par des moyens de manoeuvre (4) d'une position de repos à l'intérieur de sièges correspondants (6) dudit corps principal (2) à une position de travail à l'extérieur dudit corps principal (2), où lesdits un ou plusieurs éléments d'ancrage (3) sont poussés à l'extérieur desdits sièges correspondants (6) dudit corps principal (2) à travers une ouverture correspondante (5) quand ils sont actionnés par lesdits moyens de manoeuvre (4), **caractérisé en ce que** lesdits éléments d'ancrage (3) sont réalisés en matériel élastique, et **en ce que** lesdits sièges (6) comprennent une portion (6b) indiquée pour contenir une partie d'un élément d'ancrage correspondant (3), ladite portion (6b) ayant une profondeur (P), ou une hauteur, qui est inférieure à la longueur, ou à la hauteur (h), desdits éléments d'ancrage correspondant (3) de façon à ce que dans cette position de repos, chacun desdits un ou plusieurs éléments d'ancrage (3) est comprimé à l'intérieur de son siège correspondant (6) de façon à acquérir une première forme, et **en ce que** lesdits éléments d'ancrage (3) acquièrent une deuxième forme qui diffère de ladite première forme quand ils sont déplacés à ladite position de travail à l'extérieur dudit corps (2).

2. Dispositif selon la revendication 1), **caractérisé en ce que**, dans ladite position de repos, chacun desdits un ou plusieurs éléments d'ancrage (3) est positionné de façon à ce que son extrémité, apte à être implantée dans l'os (O), entre dans ladite ouverture (5) mais ne s'étend pas dudit corps principal (2).

3. Dispositif (200, 300) selon l'une des revendications 1) et 2), **caractérisé en ce que**, durant la sortie/rentrée desdits un ou plusieurs éléments d'ancrage (3) de/dans ledit siège (6), une surface (31, 32) dudit élément d'ancrage (3) entre en contact avec une paroi (50, 51, 52) de ladite ouverture (5) pour guider chacun desdits un ou plusieurs éléments d'ancrage (3).

4. Dispositif (200, 300) selon l'une quelconque des revendications précédentes, **caractérisé en ce que**, durant la sortie desdits un ou plusieurs éléments d'ancrage (3), chacun d'eux tourne autour d'un axe (30) disposé transversalement à l'axe longitudinal (29) dudit dispositif (1) pour faciliter leur sortie à travers ladite au moins une ouverture (5).

5. Dispositif selon l'une quelconque des revendications précédentes, **caractérisé en ce que** lesdits éléments d'ancrage (3) comprennent une tige (7) avec une première extrémité (7a) avec une portion finale pointue (48) apte à être implantée dans l'os et une deuxième extrémité (7b) dotée de moyens (9) pour relier lesdits moyens de manoeuvre (4), ladite tige étant galbée et ayant une longueur (L) et une hauteur (h).

6. Dispositif selon la revendication 5), **caractérisé en ce que** ladite tige galbée (7) est arquée et reproduit essentiellement un arc d'un circonférence, ou un arc d'une ellipse ou un arc d'une parabole.

7. Dispositif selon les revendications 5) ou 6), **caractérisé en ce que** lesdits moyens de connexion (9) comprennent une extrémité (7b) de chaque élément d'ancrage (3), ladite extrémité étant opportunément galbée pour être acueillie à l'intérieur d'une cavité correspondante (8) dans ledits moyens de manoeuvre (4).

8. Dispositif selon l'une quelconque des revendications précédentes, **caractérisé en ce que** lesdits moyens de manoeuvre (4) comprennent un premier élément (10) conçu pour coulisser axialement à l'intérieur dudit corps principal (2) relié mécaniquement à un deuxième élément (15) avec un premier filetage indiqué pour s'accoupler avec un filetage correspondant prévu sur ledit corps principal (2), ledit deuxième élément (15) étant conçu pour être tourné au moyens d'un outil (T).

9. Dispositif selon la revendication 8), **caractérisé en ce qu'**au moins une partie desdits sièges (6) sont créés longitudinalement sur la surface extérieure dudit premier élément (10) et comprennent une première portion (6a) apte à loger une extrémité (7b) dudit au moins un élément d'ancrage (3).

10. Dispositif selon l'une quelconque des revendications précédentes, **caractérisé en ce que** lesdites ouvertures (5) sont des fentes délimitées par des parois (51, 52).

11. Dispositif selon la revendication 10), **caractérisé en ce que** lesdits parois (51, 52) forment par rapport à l'axe longitudinal (29) dudit corps principal (2) un angle compris entre 72° et 68°.

12. Dispositif selon la revendication 10), **caractérisé en ce que** lesdites parois (51, 52) forment par rapport à l'axe longitudinal (29) dudit corps principal (2) un angle mesurant 35°.

13. Dispositif selon la revendication 10), **caractérisé en ce que** lesdites parois (51, 52) forment par rapport à l'axe longitudinal (29) dudit corps principal (2) un angle compris entre 145° et 165°, et préférablement mesurant 150°.

14. Dispositif selon l'une quelconque des revendications précédentes, **caractérisé en ce que** l'axe (55) desdites ouvertures (5) forme préférablement un angle aigu (B) par rapport à l'axe longitudinal (29) dudit corps principal (2).

15. Dispositif selon la revendication 14), **caractérisé en ce que** ledit angle (B) est compris entre 72° et 68°.

16. Dispositif selon la revendication 14), **caractérisé en ce que** ledit angle (B) mesure 35°.

17. Dispositif selon la revendication 14), **caractérisé en ce que** ledit angle (B) est compris entre 15° et 35° et préférablement mesure 30°.

18. Dispositif selon l'une quelconque des revendications précédentes, **caractérisé en ce que** lesdits au moins un ou plusieurs éléments d'ancrage (3) sont disposés radialement à un angle (A) mesurant environ 120° l'un de l'autre par rapport à l'axe longitudinal (9) dudit corps principal (2).

19. Dispositif selon l'une quelconque des revendications précédentes, **caractérisé en ce que** ledit corps principal (2) comprend un corps creux avec lesdites ouvertures (5) et avec une première et une deuxième extrémités (2a, 2b).

20. Dispositif selon l'une quelconque des revendications précédentes, **caractérisé en ce que** ledit corps principal (2) présente au moins une portion avec une forme externe essentiellement polygonale.

21. Dispositif selon l'une quelconque des revendications précédentes, **caractérisé en ce qu'**il comprend une coiffe (21) apte à être positionnée sur la paroi extérieure de l'os et ancrable audit os.

22. Dispositif selon l'une quelconque des revendications précédentes, **caractérisé en ce qu'**il comprend un trou passant transversal (90) indiqué pour contenir une vis d'ancrage (91) audit os et/ou un autre dispositif (1).

23. Dispositif selon la revendication 22), **caractérisé en ce que** ledit trou passant transversal (90) présente une section circulaire et/ou elliptique, et/ou polygonale, et/ou mixtiligne.
